# EUROPÄISCHE PATENTSCHRIFT

(11) **EP 0 571 457 B1**
(45) Veröffentlichungstag und Bekanntmachung des Hinweises auf die Patenterteilung: **15.01.1997**
(21) Anmeldenummer: 92904592.0
(22) Anmeldetag: 07.02.1992
(51) Int. Cl.: A61K 31/557

(54) **ILOPROST MIT WIRKUNG GEGEN CEREBRALE MALARIA**
ILOPROST EFFECTIVE AGAINST CEREBRAL MALARIA
ILOPROST A ACTIVITE ANTIPALUDIQUE CONTRE LA MALARIA CEREBRALE

(30) Priorität: 12.02.1991 DE 4104606
(43) Veröffentlichungstag der Anmeldung: 01.12.1993
(73) Patentinhaber: SCHERING AKTIENGESELLSCHAFT, 13342 Berlin (DE)
(72) Erfinder: BLITSTEIN-WILLINGER, Eveline, Dr., D-10707 Berlin (DE); SLIWA-HENLE, Karen, 2109 Melville, Johannesburg (ZA)
(86) Internationale Anmeldenummer: DE9200091
(87) Internationale Veröffentlichungsnummer: WO9213537

(56) Entgegenhaltungen:
- Dialog Information Services, File Medline, Accession Nr. 91373007, Derwent Publications Ltd, London, GB; K. SLIWA et al.: "Prevention of murine cerebral malaria by a stable prostacyclin analog"
- THE LANCET, (Index: Band 2, Juli-Dezember 1982, herausgeben von I. Munro), 11. September 1982; M.J. WESTON et al.: "Prostacyclin in falciparim malaria", Seite 609, siehe das ganze Dokument
- STN File Server, Karlsruhe, DE, Chemical Abstracts, Band 113, Nr. 7, 1990, Columbus, Ohio, US; S. PALAOGLU et al.: "Cytoprotective effect of iloprost on isloated cortical brain tissue grafts in rats", siehe Zusammenfassung Nr. 53008s
- Prostaglandins, Band 35, Nr. 5, Mai 1988; M.G. BORZEIX et al.: "Effects of new chemically and metabolically stable prostacyclin analogues (iloprost and ZK 96480) on early consequences of a transient cerebral oligemia, in the rat", Seite 653, siehe Zusammenfassung

## Beschreibung

Die vorliegende Erfindung betrifft die Verwendung von Iloprost zur Herstellung eines Arzneimittels zur Behandlung der cerebralen Malaria.

In Lancet, 1982, 609 wurde bereits über die erfolgreiche Behandlung von cerebraler Malaria, der schwersten Komplikation der Plasmodium falciparum Malaria, mit Prostacyclin (PGI₂) an einem einzigen Patienten berichtet. Außerdem erwähnt der WHO-Bericht "Severe and Complicated Malaria, second edition, Vol. 84, Suppl. 2, 1990, 1-65" 2 weitere unveröffentlichte Fälle, die ebenfalls mit Prostacyclin behandelt wurden.

Es wurde nun gefunden, daß Iloprost (I), seine 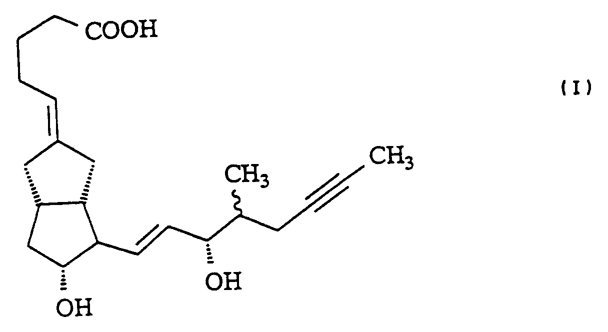 Salze mit physiologisch verträglichen Basen und sein β-Cyclodextrinclathrat in vorteilhafter Weise zur Behandlung der cerebralen Malaria geeignet sind.

Obwohl Iloprost, im Gegensatz zu PGI₂ ein stabiles Prostacyclin-Derivat, seit 1980 durch die europäische Patentanmeldung EP 11591 bekannt ist, wurde bisher kein weiteres Prostacyclin-Derivat in dieser Indikation untersucht. Es liegt daher die Vermutung nahe, daß es sich in dem publizierten Fall um eine Spontanheilung handelte.

Es wurde nun überraschend gefunden, daß Iloprost bei der cerebralen Malaria wirksam ist.

Zur Salzbildung von Iloprost sind anorganische und organische Basen geeignet, wie sie dem Fachmann zur Bildung physiologisch verträglicher Salze bekannt sind. Beispielsweise seien genannt: Alkalihydroxide, wie Natrium- und Kaliumhydroxids, Erdalkalihydroxide, wie Calciumhydroxid, Ammoniak, Amine, wie Äthanolamin, Diäthanolamin, Triäthanolamin, N-Methylglucamin, Morpholin, Tris(hydroxymethyl)-methylamin usw.
Die β-Cyclodextrinclathrat-Bildung erfolgt entsprechend EP 259468.

Die Herstellung von Iloprost wird detailliert in EP 11591 beschrieben.

In EP 11591 werden für die dort beschriebenen Carbacyclinderivate folgende pharmakologische Eigenschaften beschrieben:

Senkung des peripheren arteriellen und koronaren vaskulären Widerstandes, Inhibierung der Thrombozytenaggregation und Auflösung von Plättchenthromben, myocardiale Zytoprotektion; Senkung des systemischen Blutdruckes ohne zugleich Schlagvolumen und koronare Durchblutung zu senken; Behandlung von Schlaganfall, Prophylaxe und Therapie koronarer Herzerkrankungen, koronarer Thrombose, des Herzinfarkts, peripherer Arterienerkrankungen, Arteriosklerose und Thrombose, Therapie des Schocks, Inhibierung der Bronchokonstriktion, Inhibierung der Magensäuresekretion und Zytoprotektion der Magen- und Darmschleimhaut; antiallergische Eigenschaften, Senkung des pulmonalen vasculären Widerstandes und des pulmonalen Blutdruckes, Förderung der Nierendurchblutung, Anwendung an Stelle von Heparin oder als Adjuvans bei der Dialyse oder Hämofiltration, Konservierung von Blutplasmakonserven, besonders von Blutplättchenkonserven, Inhibierung von Geburtswehen, Behandlung von Schwangerschaftstoxikose, Erhöhung der zerebralen Durchblutung und Antiproliferation.

Die Behandlung der cerebralen Malaria durch Iloprost wird nicht beschrieben und steht auch in keinem direkten Zusammenhang mit den in EP 11591 beschriebenen sonstigen pharmakologischen Eigenschaften.

Die Dosis von Iloprost ist 1-1500µg/kg/Tag, wenn sie am menschlichen Patienten verabreicht wird. Die Einheitsdosis für den pharmazeutischen akzeptablen Träger beträgt 0,01-100 mg.

Die Dosierung einer i.v.-Verabreichung als Dauerinfusion in üblichen wässrigen Lösungsmitteln, z.B. 0,9%iger NaCl-Lösung, erfolgt vorzugsweise in Dosierungen zwischen 0,1 ng/kg/min und 0,1µg/kg/min.

Der Wirkstoff soll in Verbindung mit den in der Galenik bekannten und üblichen Hilfsstoffen zur Herstellung von Mitteln gegen cerebrale Malaria dienen.

Die gegen cerebrale Malaria wirkende Verbindung wird in an sich bekannter Weise mit den an sich bekannten Hilfs-und Trägerstoffen in eine galenische Formulierung gebracht.

### Beispiele

### Mausmodell für cerebrale Malaria

CBA/CA-Mäuse entwickeln nach Inokulation mit 1x10⁶ parasitierten Erythrozyten (Plasmodium Berghei ANKA) i.p. zu 80 - 90% zwischen dem 7ten und 14ten Tag das Krankheitsbild der cerebralen Malaria mit neurologischen Symptomen (Verlangsamung, Paralyse, Ataxie, Konvulsionen), an denen sie innerhalb weniger Stunden (1-5 Std.) versterben. Zu diesem Zeitpunkt liegt eine nur geringgradige Anämie bei einer Parasitamie von nur 8-10% vor. Die überlebenden Mäuse sterben nach 3-4 Wochen an schwerer Anämie mit einer Parasitämie von über 50%. Grau et al. (Science, Vol. 237 (1987)) haben die entscheidende Rolle von TNF an der Pathogenese und Prognose dieser Krankheit durch folgende Ergebnisse belegen können:
a) Zum Zeitpunkt des Auftretens cerebraler Komplikationen zeigen die für cerebrale Malaria empfänglichen Mäuse deutlich erhöhte Serum TNF-Spiegel.
b) Passive Immunisierung gegenüber Maus-TNF reduziert signifikant das Auftre ten cerebraler Malaria bei mit Plasmodium burghei infizierten CBA/CA-Mäuse.
c) Die Behandlung mit anti-TNF-Antikörpern verhindert ebenfalls die histopathologischen Veränderungen, die für die cerebrale Malaria bedeutsam sind.

### Beispiel 1

Durch intraperitoneale Injektion von 2 % Starkelösung wird in NMRI-Mäusen eine lokale sterile Entzündung gesetzt. Nach 3-5 Tagen werden die Tiere getötet und die Makrophagen gewonnen.
In vitro Versuche zeigen, daß Iloprost die durch hitzelabiles Antigen, das aus Plasmodium burghei gewonnen wurde, induzierte TNF-Produktion der NMRI-Mause-Peritonealmakrophagen dosisabhängig hemmt.

Die nicht adhärenten Zellen werden abgetrennt.
Zur Aktivierung der Makrophagen wird hitzestabiles lösliches Antigen, welches mit P.b. ANKA parasitierter muriner Erythrozythen präpariert wird (2) benutzt.

Als TNF-Assay wird die TNF-sensitive Zellinie WEHI 164 (im Handel zu erhalten) verwendet. Das Ausmaß der Zellyse von WEHI 164 ist proportional zur vorhandenen Menge an TNF. In 96Napfflachbodenmikrotiter-Platten werden die Kulturüberstände und Seren in einer Verdünnungsreihe austitriert. Eine Titrationsreihe mit TMU TNF wird als Standard benutzt.

Die Zahl der überlebenden Zellen wird anhand des kolorimetrischen MTT-Testes bestimmt.

Die Berechnung wird durch den Vergleich mit der Standardtitrationsreihe von TMU TNF mittels der Probitanalyse durchgeführt.

Der Test erlaubt eine Bestimmung von bis zu 0,5 U/ml TNF. Durch Zugabe eines anti TNF-Antiserums kann zwischen TNFα und TNFβ differenziert werden.

### Beispiel 2

### Einsatz von Iloprost im Tiermodell

15 Mäuse werden durch die intraperitoneale Gabe von 1x10⁶ parasitierter Erythrozyten infiziert. Gleichzeitig mit der Infektion erfolgt bei 6 Mäusen die Gabe von 1 µg Iloprost (100 µl s.c.). Diese Behandlung wird täglich um die gleiche Zeit bis zum 10. Tag fortgeführt.

Die 9 Mäuse der Kontrollgruppe werden wie oben beschrieben behandelt, erhalten aber an Stelle von Iloprost 100 µl NaCl. Die Parasitämie wird täglich bestimmt (Blutausstrich, Giemsa Färbung).
Beide Gruppen erreichen eine 10%ige Parasitämie am Tag 10 post infectionem.

Von der Kontrollgruppe sterben zwischen dem 8. und 14. Tag 8 von 9 Mäusen mit vorangegangenen cerebralen Erscheinungen. Die Mäuse der Iloprost-Gruppe zeigen zu diesem Zeitpunkt keine cerebralen Symptome.
Die letzte Maus der Kontrollgruppe sowie die Mäuse der Iloprostgruppe, sterben zwischen dem 24, und 30. Tag nach der Infektion an den Folgen der schweren Anämie.

### Beispiel 3

Die Serum TNF-Spiegel der unbehandelten und der Iloprost-behandelten Mäuse werden untersucht.
Iloprost hemmt signifikant die TNF-Spiegel im Serum auch noch 4 Tage nach der letzten Injektion.

### Literatur

1. G.Grau, L.Fajardo, P.Piguet, B.Allet, P.Lambert, P.Vassalli, Science, Vol.237(1987)
2. J.Taverne, C.Bate, Parasite Immunology 1990, 12, 33-43

## Patentansprüche

1. Verwendung von Iloprost zur Herstellung eines Arzneimittels zur Behandlung der cerebralen Malaria.

## Claims

1. Use of iloprost in the preparation of a medicament for the treatment of cerebral malaria.

## Revendications

1. Utilisation de l'iloprost pour la préparation d'un médicament pour le traitement de malaria cérébrale.
